(19) Europäisches Patentamt
European Patent Office
Office européen des brevets

(11) Publication number : **0 137 668 B2**

(12) **NEW EUROPEAN PATENT SPECIFICATION**

(45) Date of publication of the new patent specification : **17.03.93 Bulletin 93/11**

(51) Int. Cl.⁵ : **A61K 31/19**, // C07C57/30, C07C57/58

(21) Application number : **84305661.5**

(22) Date of filing : **20.08.84**

(54) **Ibuprofen, flurbiprofen and their therapeutic use.**

(30) Priority : **31.08.83 US 528272**

(43) Date of publication of application : **17.04.85 Bulletin 85/16**

(45) Publication of the grant of the patent : **17.01.90 Bulletin 90/03**

(45) Mention of the opposition decision : **17.03.93 Bulletin 93/11**

(84) Designated Contracting States : **BE CH DE FR GB IT LI NL SE**

(56) References cited :
EP-A- 0 070 714
GB-A- 1 550 139
US-A- 4 346 108

(73) Proprietor : **THE UPJOHN COMPANY**
**301 Henrietta Street**
**Kalamazoo, Michigan 49001 (US)**

(72) Inventor : **Wechter, William Julius**
**c/o The Upjohn Company 301 Henrietta Street**
**Kalamazoo Michigan 49001 (US)**

(74) Representative : **Perry, Robert Edward et al**
**GILL JENNINGS & EVERY, Broadgate House,**
**7 Eldon Street**
**London EC2M 7LH (GB)**

EP 0 137 668 B2

## Description

This invention relates to flurbiprofen and its therapeutic use.

Periodontal disease is a broad term used to describe those diseases which attack the gingiva and the underlying alveolar bone housing the teeth. At present, the treatment of periodontal disease is one of the most difficult and costly dental problems. The two most common periodontal diseases are chronic gingivitis, i.e. inflammation of the gingiva, and chronic destructive periodontitis, which results in progressive resorption of alveolar bone, increasing mobility of the teeth, and loss of the teeth at the advanced stage. It is known that periodontal disease is caused ultimately by bacteria which accumulate on the teeth and underneath the gingiva. Also, it is known that the disease process of chronic destructive periodontitis has major inflammatory and immunopathologic components which may indicate that an individual's response to the bacteria may be destructive in itself rather than protective.

The prevention and treatment of chronic destructive periodontal disease has varied little over the past two decades and consists primarily of eliminating subgingival calculus, overhanging fillings, soft tissue, infra-bony pockets and occlusal trauma and establishing good oral hygiene-and a periodontal environment hat is easily kept clean by the patient. The therapy is not only long and expensive, but it never truly ends. Periodontal surgery must frequently be repeated and, most importantly, there are no good data that clearly demonstrate the long-term efficacy of such treatment with regard to arresting alveolar bone loss and preserving the natural dentition. Clearly, new modes of therapy are sorely needed to substitute and augment some of our current procedures, or to supplement them in order to make them more effective or available to more people. Alveolar bone loss or resorption accompanying tooth extraction represents another serious dental problem for which no satisfactory prophylactic treatment has been available to date.

As reported in J. Periodontal Res. *16*, 659-665 (1981), R. C. Williams *et al.* have demonstrated in beagle dogs that tetracycline administration is effective over a 12-month period in decreasing the rate of alveolar bone loss. Also, the results of a retrospective study reported by R. S. Feldman *et al.*, in J. Clin. Peridontal. *10*, 131-136 (1983), suggest that the chronic ingestion of aspirin or aspirin together with indomethacin resulted in the inhibition of alveolar bone loss. Other references which report the effect of indomethacin on bone resorption associated with periodontal disease include M. Weaks-Dybvig *et al.*, J. of Periodontal Research *17*, 90-100 (1982); S. Nyman *et al.*, J. Periodontol. *50*, 450-461 (1979); and J. J. Lasfargues *et al.*, J. of Periodontal Research *18*, 110-117 (1983). I. M. Waite *et al.*, J. of Periodontal Research *16*, 100-108 (1981), report on the periodontal status of subjects receiving non-steroidal antiinflammatory drugs. EP-A-0068563 describes compounds which modify the balance between bone production and bone resorption, but there is no indication that these compounds are useful in treating periodontitis.

Flurbiprofen is 2 - fluoro - $\alpha$ - methyl[1,1' - biphenyl]- 4 - acetic acid and is prepared as described in US-A-3755427.

Flurbiprofen is a known anti-inflammatory agent that possesses analgesic and antipyretic activities and has been indicated as useful in patients with symptoms of rheumatoid arthritis and osteoarthritis.

The method of the present invention comprises the use of flurbiprofen or a pharmaceutically acceptable salt or lower alkyl $C_{1-6}$ ester of said compound for the manufacture of a medicament for the inhibition or prevention of alveolar bone resorption. Pharmaceutically acceptable salts of flurbiprofen useful in practicing the present invention are alkali metal salts such as the potassium or sodium salt, alkaline earth salts such as calcium or magnesium, amine salts such as t-butyl amine or aluminium salts. Lower alkyl $C_{1-4}$ esters of flurbiprofen useful in practicing the present invention include the methyl, ethyl, n-propyl, isopropyl, n-butyl, tert-butyl pentyl, esters. In practicing the method of the present invention flurbiprofen or a salt or ester thereof is administered orally, topically, or bucally to the patient to be treated which is any mammal such as, dogs cats, horses, various cattle, rats, mice or humans. Although the compounds are effective in preventing or inhibiting alveolar bone loss when administered intravenously this obviously is a less preferred method of administration. However, the injection of the compounds directly into the gum or by subdermal implantation represent effective means of administering flurbiprofen to achieve inhibition or prevention of alveolar bone loss. The compounds may also be administered transdermally. Suitable forms of oral administration include tablets, capsules, pills, powders, granules, solutions or suspensions. Sterile aqueous solutions or suspensions are also suitable for injection in practicing the method of the present invention. Suitable topical forms of administration include gels, pastes, or an adhesive patch containing the compound. Suitable formulations for buccal administration include slow dissolving tablets, troches, chewing gum, a gel, paste or powder, e.g., to be applied to the gums, including a denture adhesive.

Surprisingly, very low non-anti-inflammatory quantities of flurbiprofen are effective for the prophylaxis or treatment of alveolar bone resorption.

The quantity of flurbiprofen effective in preventing or inhibiting alveolar one resorption. varies from 0.05 to 6 mg/kg per day and preferably from 1 to 4 mg/kg per day. For example unit doses of 50 mg given 2 times per day is suitable in practicing the present invention.

Compositions suitable for use in the present invention are formulated using conventional pharmaceutical carriers. For example, solid dosage forms typically will contain lubricants such as stearic acid or magnesium stearate and fillers such as lactose, sucrose, cornstarch, or perhaps disintegrating agents such as alginic acid. Injectable solutions or suspensions may contain sterile liquids such as water or oils with surfactants as needed. Representative examples of compositions useful in practicing the use of the present invention are set forth hereinbelow.

The medicament used in the present invention is for the prevention or inhibition of alveolar bone loss or resorption. The alveolar bone loss may be that associated with chronic destructive periodontitis. Thus the present invention provides in essence a use of the above cited compound for the manufacture of a medicament for treating chronic destructive periodontitis in that the alveolar bone resorption associated therewith is inhibited or prevented. The alveolar bone loss or resorption may be that associated with the extraction of a tooth or teeth as is, e.g., so dramatically demonstrated in whole mouth tooth extractions. The method of the present invention is useful in preventing or inhibiting the apparent shrinkage of the gum line following tooth extraction.

The patient to be treated is one which possesses signs of alveolar bone resorption. Such patients include those having periodontitis as evidenced by the condition of the gingival tissue of said patients. The gingiva of the patient having periodontitis is characterized by having severe inflammation with marked redness and edema, ulceration, and a tendency to bleed spontaneously. Additionally the presence of subgingival calculus and an accumulation of soft deposits within the gingival pockets are characteristic of more advanced stages and of course a loosening of the teeth marks a severe loss of alveolar bone. Additionally the patient to be treated is one who has one or more teeth extracted or significantly altered as, e.g., by root canal therapy. More generally reports indicate periodontal disease is endemic in the population over age 35 in the United States and thus individuals in this class would benefit from the invention described herein.

The utility of the present invention is demonstrated in an 18-month study using the beagle dog model as generally described in J. Periodontal Res. 16, 659-665 (1981). The 18-month study period included a six-month pretreatment period in which baseline data were gathered and a 12-month treatment period. Two groups of six dogs each serve as control and test groups. At the beginning of the treatment period one half of the mouth of each dog received conventional surgical periodontal treatment followed by regular tooth brushing and prophylaxis. Throughout the treatment period each test group dog received orally 0.02 mg/kg per day of flurbiprofen except on the first four days of treatment when 0.2 mg/kg per day of compound was administered. The control group received no test compound. The loss of alveolar bone for each group was determined radiographically at 3, 6, 9 and 12 month intervals. The results are set forth in the following Table I and indicate that flurbiprofen dramatically reduced or arrested the rate of bone loss over the 12-month period.

### TABLE I
Comparison of the overall six months pretreatment period rate of bone loss with the rate of
bone loss at 3, 6, 9, and 12 months of the treatment period

Untreated control dogs

| | | | |
|---|---|---|---|
| Non-surgically treated | Pretreatment rate | .78±.10 | |
| | 3 month treatment | .93±.13 | p<.32 |
| | 6 month treatment | 1.33±.21 | p<.004 |
| | 9 month treatment | .63±.27 | p<.363 |
| | 12 month treatment | .73±.18 | p<.472 |
| Surgically treated | Pretreatment rate | .39±.07 | |
| | 3 month treatment | 1.40±.1 | p=0 |
| | 6 month treatment | .59±.18 | p<.14 |
| | 9 month treatment | .29±.20 | p<.001 |
| | 12 month treatment | .23±.12 | p<.28 |

Flubiprofen treated dogs

| | | | |
|---|---|---|---|
| Non-surgically treated | Pretreatment rate | .69±.13 | |
| | 3 month treatment | .12±.09 | p<.001 |
| | 6 month treatment | .32±.25 | p<.08 |
| | 9 month treatment | .09±.25. | p<.02 |
| | 12 month treatment | .13±.16 | p<.003 |
| Surgically treated | Pretreatment rate | .68±.14 | |
| | 3 month treatment | 0±.16 | p<.003 |
| | 6 month treatment | .20±.25 | p<.05 |
| | 9 month treatment | .25±.21 | p<.04 |
| | 12 month treatment | 0±.14 | p=0 |

The following examples provide illustrative compositions useful in practicing the present invention.

Example 1

Mouthwash composition

| | | |
|---|---|---|
| Flurbiprofen | 5.00 | gm |
| Methylparaben | 0.75 | gm |
| Propylparaben | 0.25 | gm |
| Saccharin | 1.50 | gm |
| Ascorbic acid | 1.00 | gm |
| Orange oil flavor | 1.00 | gm |
| FDC orange dye | 0.75 | gm |
| Sucrose | 200.00 | gm |
| Deionized water, qs | 1000.00 | ml |

The saccharin, ascorbic acid, orange flavor, dye, and parabens are dissolved in 600 ml of deionized water. The flurbiprofen is mixed with the sucrose and the mixture dispersed in the aqueous solution. Sufficient deionized water is added to make 1000 ml of the composition, containing 5 mg per ml or 25 mg per teaspoonful of active ingredient.

Example 2

Chewing gum

Pieces of uncoated chicle chewing gum of tablet-like shape weighing about one gram each are used as starters for the coating process. A coating of sucrose is first applied, using a syrup (80% sucrose solution) and the usual coating pan. A finely powdered highly hydrogenated castor oil is dusted on the sucrose covered starters. The second coating step is the application of a dispersion of micronized flurbiprofen and benzocaine (10:1) in absolute ethanol. Successive applications are made until 10 mg of flurbiprofen and 1 mg of benzocaine have been deposited on each piece. Thereafter, a second dusting of the hydrogenated castor oil is applied. A final finishing coating is applied of sucrose solution containing flavor and color. Advantageously a polishing coat of wax is added.

The foregoing chewing gum composition is chewed in the mouth to provide the slow release of flurbiprofen for the topical treatment.

Example 3

Toothpaste

| | |
|---|---|
| Flurbiprofen | 10.0% |
| Magnesium aluminum silicate | 1.0% |
| Dicalcium phosphate | 47.0% |
| Sodium carboxymethylcellulose | 0.5% |
| Mint flavor | 4.0% |
| Sodium lauryl sulfate | 2.0% |
| Benzoic acid | 0.1% |
| Water | 35.4% |

Add all ingredients slowly to the water while stirring and then pass the mixture through a roller mill.

Example 4

Hard gelatin capsules

One thousand two-piece hard gelatin capsules for oral use, each capsule containing 50 mg of flurbiprofen are prepared from the following:

| | |
|---|---|
| Flurbiprofen | 50 gm |
| Lactose | 100 gm |
| Corn starch | 20 gm |
| Talc | 20 gm |
| Magnesium stearate | 2 gm |

The flurbiprofen (finely divided by means of an air micronizer) is added to the other finely powdered ingredients, mixed thoroughly and then encapsulated in the usual manner.

Using the procedure above, capsules are similarly prepared containing flurbiprofen in 25, 75, and 100 mg amounts by substituting 25, 75 and 100 gm of flurbiprofen for the 50 gm used above.

Example 5

Soft gelatin capsules

One-piece soft gelatin capsules for oral use, each containing 25 mg of flurbiprofen (finely divided by means of an air micronizer) are prepared by first suspending the compound in 0.5 ml of corn oil to render the material capsulatable and then capsulating in the above manner.

Example 6

Tablets

One thousands tablets, each containing 100 mg of flurbiprofen are prepared from the following types and amounts of ingredients:

| | |
|---|---|
| Flurbiprofen micronized | 100 gm |
| Lactose | 75 gm |
| Corn starch | 50 gm |
| Magnesium stearate | 4 gm |
| Light liquid petrolatum | 5 gm |

The flurbiprofen (finely divided by means of an air micronizer) is added to the other ingredients and then thoroughly mixed and slugged. The slugs are broken down by forcing through a number sixteen screen. The resulting granules are then compressed into tablets, each tablet containing 100 mg of flurbiprofen.

Using the procedure above, tablets are similarly prepared containing flurbiprofen in 25 mg and 50 mg amounts by substituting 25 gm and 50 gm of flurbiprofen for the 100 gm used above.

Example 7

Oral suspension

One thousand ml of an aqueous suspension for oral use, containing in each teaspoonful (5 ml) dose, 100 mg of flurbiprofen, sodium salt is prepared from the following types and amounts of ingredients:

| | |
|---|---|
| Flurbiprofen, sodium salt micronized | 20 gm |
| Citric acid | 2 gm |
| Benzoic acid | 1 gm |
| Sucrose | 400 gm |
| Cellulose, microcrystalline | 10 gm |
| Lemon oil | 2 gm |
| Polysorbate 80 | 5 gm |
| Deionized water, qs | 1000 ml |

The citric acid, benzoic acid, sucrose, cellulose, lemon oil and polysorbate 80 are dispersed in sufficient water to make 850 ml of suspension. The flurbiprofen sodium salt (finely divided by means of an air micronizer) is stirred into the syrup until uniformly distributed. Sufficient water is added to make 1000 ml.

Example 8

A sterile aqueous solution for parenteral (i.v) injection, containing in one liter, 150 mg of flurbiprofen, sodium salt is prepared from the following types and amounts of ingredients:

| | |
|---|---|
| Flurbiprofen sodium salt | 150 mg |
| Water for injection, qs | 1000 ml |

To the sterile solution is added sterilized flurbiprofen, sodium salt and filled into sterile containers sealed.

Example 9

Oral topical paste

| | |
|---|---|
| Flurbiprofen, aluminum salt micronized | 10.0% |
| Sodium carboxymethylcellulose | 3.0% |
| Pectin | 3.0% |
| Gelatin | 3.0% |
| Sodium saccharin | 0.5% |
| Polysorbate 80 | 0.5% |
| Cherry flavor | 0.1% |
| Water | 79.9% |

Heat water to 50°C and add, while stirring, polysorbate 80, and all other ingredients. Pass through a colloid mill, then stir until cool.

Example 10

Sustained release tablet

The following formulation is representative of a 100 mg sustained release formulation useful in practicing the present invention. The 100 mg sustained release formulation may be used in place of other dosage unit forms which contain 50 mg of active ingredients for administration, for example twice a day.

| | |
|---|---|
| (a) Flurbiprofen milled | 100.0 mg |
| (b) Lactose USP hydrous bolted | 95.0 mg |
| (c) Pregelatinized starch NF | 10.5 mg |
| (d) Purified water USP, q.s. | |
| (e) Methocel K 15 M premiuam | 95.0 mg |
| (f) Colloidal silicon dioxide NF | 1.0 mg |
| (g) Magnesium stearate NF | 2.5 mg |
| (h) Hydroxypropyl methylcellulose 2910 USP 15 CPS | 10.0 mg |
| (i) Film coat concentrate white FC-100-PC | 2.0 mg |
| (j) Purified water USP, q.s. ad | |

Ingredients (a) to (d) are blended, granulated, wet screened, and dried, then mixed with ingredients (e) to (g) and coated with ingredients (h) to (i).


**Claims**

1. Use of flurbiprofen or a $C_{1-6}$ alkyl ester or pharmacologically-acceptable salt of flurbiprofen, for the manufacture of a medicament for preventing or inhibiting alveolar bone resorption.

2. Use of flurbiprofen aluminium salt according to claim 1.

3. Use according to claim 1 or claim 2, in which the medicament is suitable for oral administration.

4. Use according to claim 1 or claim 2, in which the medicament is suitable for topical administration.

5. Use according to claim 1 or claim 2, in which the medicament is suitable for buccal adminsitration.


**Patentansprüche**

1. Gebrauch von Flurbiprofen oder eines $C_{1-6}$ Alkylesters oder pharmakologisch annehmbaren Salzes von Flurbiprofen zur Fertigung eines Medikamentes zwecks Verhütung oder Hemmung von Alveolarknochenresorption.

2. Gebrauch von Flurbiprofenaluminiumsalz im Einklang mit Anspruch 1.

3. Gebrauch im Einklang mit Anspruch 1 oder Anspruch 2, wobei das Medikament für mündliche Verabreichung geeignet ist.

4. Gebrauch im Einklang mit Anspruch 1 oder Anspruch 2, wobei das Medikament für topische Verabreichung geeignet ist.

5. Gebrauch im Einklang mit Anspruch 1 oder Anspruch 2, wobei das Medikament für bukkale Verabreichung geeignet ist.


**Revendications**

1. Utilisation du flurbiprofène ou d'un ester alkylique en $C_1$ à $C_6$ ou sel pharmacologiquement acceptable du flurbiprofène, pour la production d'un médicament destiné à empêcher ou inhiber la résorption du tissu osseux alvéolaire.

2. Utilisation du sel d'aluminium du flurbiprofène, suivant la revendication 1.

3. Utilisation suivant la revendication 1 ou la revendication 2, dans laquelle le médicament convient pour l'administration par voie orale.

4. Utilisation suivant la revendication 1 ou la revendication 2, dans laquelle le médicament convient pour l'administration topique.

5. Utilisation suivant la revendication 1 ou la revendication 2, dans laquelle le médicament convient pour l'administration au niveau buccal.